# EUROPÄISCHE PATENTSCHRIFT

(11) **EP 0 320 791 B1**
(45) Veröffentlichungstag und Bekanntmachung des Hinweises auf die Patenterteilung: **03.11.1993**
(21) Anmeldenummer: 88120508.2
(22) Anmeldetag: 08.12.1988
(51) Int. Cl.: A61C 8/00

(54) **Fingerschlüssel**
Finger wrench
Clé à doigt

(30) Priorität: 12.12.1987 DE 8716414 U
(43) Veröffentlichungstag der Anmeldung: 21.06.1989
(73) Patentinhaber: Bauer, Ernst, Dr., D-61231 Bad Nauheim (DE)
(72) Erfinder: Schuster, Wilhelm, DE-6000 Frankfurt/Main 90 (DE)
(74) Vertreter: Fuchs Mehler Weiss

(56) Entgegenhaltungen:
- DE-U- 8 607 407
- FR-A- 1 478 557
- FR-A- 2 468 352
- US-A- 2 347 567
- US-A- 4 547 157

## Beschreibung

Die Erfindung betrifft eine Eindrehanordnung zum Eindrehen von Implantatschrauben in Kieferknochen und zum Ausrichten von schräg stehenden, an den Implantatschrauben angeformten Implantatpfosten gemäß Anspruch 1.

Das Eindrehen von Implantatschrauben in Kieferknochen erfordert Sorgfalt und Fingerspitzengefühl und kann wegen des diffizilen Arbeitens nicht ohne weiteres von Hand durchgeführt werden. Bekannt sind hierfür komplizierte Gelenkschlüssel, Winkelschlüssel oder Ratschen, die jedoch nur mit großer Erfahrung und mit Geschick zuverlässig bedient werden können. Noch schwieriger ist aber das Ausrichten eines schräg stehenden Implantatpfostens, der an der Implantatschraube angeformt ist und nach dem Einlegen des Implantats in der Regel ausgerichtet werden muß, um mit den Restzähnen parallel und/oder zum Kieferkamm lotrecht angeordnet zu sein. Das Ausrichten schräg stehender Implantatpfosten geschieht bisher mit Hilfe einer Zange. Dabei ist es aber kaum zu vermeiden, daß die Oberfläche des Implantatpfostens beschädigt wird. Ferner ist eine axial senkrechte Position zur Bißstellung höchstens zufällig erreichbar.

Die US-A-4 547 157 zeigt lediglich eine Eindrehhilfe, die nur kraftschlüssig auf das Implantat einwirkt, also keinen exakten Formschluß mittels eines Vierkants aufweist, der ein Verrutschen der Eindrehhilfe sicher verhindert.

Desgleichen ist auch keine einfache Hebelanordnung vorgesehen, mit der das Implantat eingedreht werden könnte.

Die FR-A-1 478 557 zeigt die übliche Ratschenanordnung, wie sie beispielsweise beim Eindrehen von Sechskantschrauben zum Einsatz kommt. Eine derartige Ratschenanordnung ist zum einen äußerst unhandlich, um im Mundbereich angewendet werden zu können, und andererseits auch technisch zu aufwendig und damit zu voluminös, so daß eine solche Anordnung aufgrund des Raumbedarfs im begrenzten Mundbereich nicht angewendet werden kann.

Aufgabe der Erfindung ist es, eine Eindrehanordnung der eingangs genannten Art zu schaffen, die bei einfacher Ausführung und Anpassung an den Implantatpfosten einer Implantatschraube leicht und sicher zu handhaben ist und ein einwandfreies Ausrichten des Implantatpfostens gewährleistet.

Diese Aufgabe wird durch die Eindrehanordnung gemäß Anspruch 1 gelöst.

Zweckmäßige Weiterbildungen sind in dem Unteranspruch gekennzeichnet.

Mit einem solchen Fingerschlüssel ist das Arbeiten beim Einschrauben und Ausrichten eines Schraubenimplantats einfach und sicher. Der Innenkonus der Bohrung im Schaft ist der Kontur eines Implantatpfostens angepaßt.In der Regel werden Schraubenimplantate verwendet, bei denen an einem mit Gewinde versehenen Implantatkörper ein konischer Implantatpfosten zur Aufnahme einer Primär- oder einer Subkonstruktion angeformt ist. Das äußere freie Ende des Implantatpfostens ist als Vierkant ausgebildet, auf den ein Schlüssel-Vierkant aufgesteckt werden kann. Einem solchen Implantatpfosten ist der Fingerschlüssel angepaßt und zum Einschrauben des Implantats und zum Ausrichten des Implantatpfostens vorgesehen. Der Innenkonus der Sackbohrung des Schaftes des Fingerschlüssels wird auf den Konus des Implantatpfostens so weit aufgesteckt, daß der Vierkant am Ende der Innenbohrung auf dem Vierkant des Pfostens fest sitzt. Der Fingerschlüssel kann nun mit Hilfe des Rändelrades von Hand bedient werden. Zweckmäßig ist aber ein Hebelarm vorgesehen, der seitlich mindestens einen abstehenden, vorzugsweise zwei abstehende Zapfen aufweist, die mit den Ausnehmungen auf der Oberseite des Rändelrades des Fingerschlüssels zusammenwirken. Durch diesen Verlängerungshebel wird die aufzuwendende Biegekraft minimiert. Das Eindrehen der Implantatschraube wird erleichtert und das Ausrichten des Implantatpfostens kann dank der Hebelführung des aufgesteckten Fingerschlüssels und dabei Biegen des Implantathalses in der Biegezone mit äußerster Genauigkeit durchgeführt werden. Während des Biegevorganges ist die Parallelität mit den Restzähnen und/oder die Lotrechte mit dem Kieferkamm leicht kontrollier- und vergleichbar. Ein Beschädigen des Implantatpfostens ist nicht zu befürchten, da der Fingerschlüssel den Pfosten mit Paßsitz umgreift und führt.

Die Neuerung wird anhand eines in den Zeichnungen dargestellten Ausführungsbeispiels näher erläutert. Es zeigen:
Fig. 1 eine Draufsicht auf die Oberseite des Rändelrades eines Fingerschlüssels und
Fig. 2 eine Seitenansicht im Schnitt entlang der Linie A-B in Fig. 1 gesehen.

Der Fingerschlüssel 1 besteht aus einem Schaft 2 und einem an diesem angeformten Rändelrad 5.

Am freien Ende des Schaftes 2 ist eine axial geführte Sackbohrung mit einem nach außen sich erweiternden Konus 3 vorgesehen, der am Innenende der Sackbohrung in eine Vierkantbohrung 4 übergeht. Der Konus 3 der Sackbohrung weist eine der Konizität eines genormten Implantatpfostens entsprechende Winkelöffnung auf und die Vierkantbohrung 4 in der Sackbohrung ist einer Vierkantform des oberen Endes des Implantatpfostens angepaßt, um mit Paßsitz aufgesetzt werden zu können. Damit ist ein für das Eindrehen und Ausrichten eines Schraubenimplantats einfaches aber äußerst wirksames Instrument geschaffen, das sich sowohl bei den Arbeiten des Zahnarztes als auch für das Empfinden des Patienten günstig auswirkt.

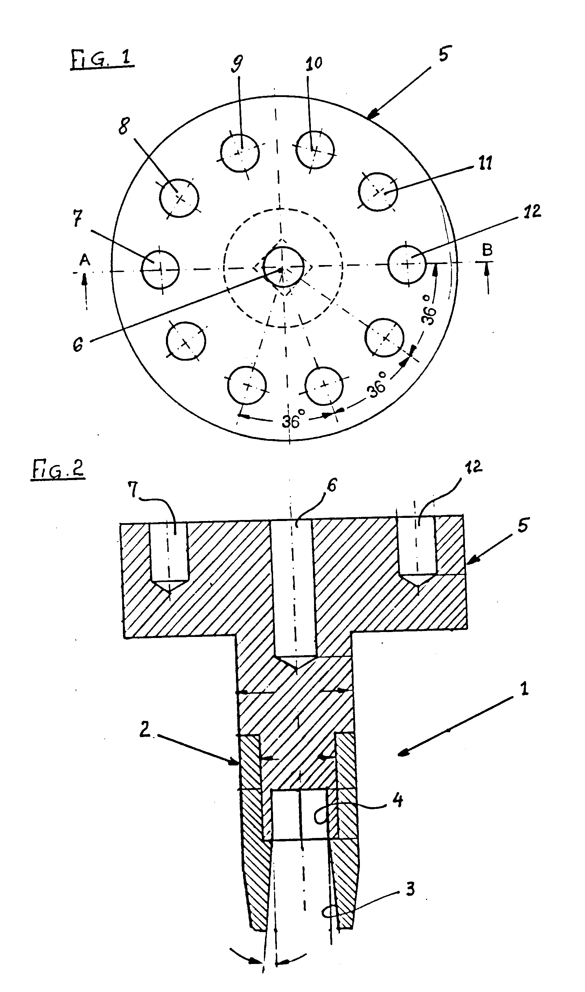

Der Gebrauch des Fingerschlüssels wird mit einem entsprechend angepaßten Hebel optimiert. Hierfür ist auf der Oberseite des Rändelrades 5 mindestens eine Zentralausnehmung 6 vorgesehen, die zweckmäßig als Vierkant ausgeführt ist, um einen seitlich abstehenden Zapfen eines Hebelarmes (nicht dargestellt) kraftschlüssig aufzunehmen. Besonders präzise arbeitet ein Fingerschlüssel 1, der neben der Zentralausnehmung 6 auf der Oberseite des Rändelrades 5 noch weitere Ausnehmungen aufweist. Solche Ausnehmungen werden in gleichmäßigem radialen Abstand von der Zentralausnehmung 6 angeordnet. In Fig. 1 sind solche Ausnehmungen 7 bis 12 numeriert, gezeigt sind insgesamt zehn Ausnehmungen, die jeweils in einem Winkelabstand von 36° voneinander angeordnet sind. Diese Aufteilung mit der Möglichkeit, zwei entsprechend am Hebelarm angeordnete abstehende Zapfen in die Zentralausnehmung 6 einerseits und in eine der Ausnehmungen 7...... andererseits einzustecken, gibt dem Zahnarzt ein Instrument, das er präzise bedienen und dabei präzise die erforderlichen Arbeiten zum Einlegen eines Schraubenimplantats durchführen kann.

Der Hebel bzw. Hebelarm mit den seitlich abstehenden Zapfen ist nicht dargestellt, da Ausführungen hierfür dem Fachmann geläufig sind.

## Patentansprüche

1. Eindrehanordnung zum Eindrehen von Implantatschrauben in Kieferknochen und zum Ausrichten von schräg stehenden, an den Implantatschrauben angeformten Implantatpfosten mit einem Fingerschlüssel, aufweisend einen Schaft (2) mit einer Sackbohrung, die einen sich nach außen erweiternden Konus (3), der an seinem Innenende in eine Vierkantbohrung (4) übergeht, und an dem der Öffnung des Konus (3) gegenüberliegenden Ende des Schaftes (2) ein Rändelrad (5) aufweist, und mit einem Hebelarm, mit dem der Fingerschlüssel bei kraftschlüssiger Aufnahme betätigbar ist.

2. Eindrehanordnung nach Anspruch 1, dadurch gekennzeichnet, daß in der Oberseite des Rändelrades (5) eine Zentralausnehmung (6) und in gleichmäßigem radialem Abstand von der Zentralausnehmung (6) mehrere weitere Ausnehmungen (7, 8, ...) angeordnet sind und der Hebelarm zwei voneinander beabstandete Zapfen aufweist, von denen der erste Zapfen mit der Zentralausnehmung (6) und der zweite Zapfen mit einer der weiteren Ausnehmungen (7, 8, ...) kraftschlüssig verbindbar ist.

## Claims

1. Screw-in arrangement for implant screws to be screwed in jaw bones and for the positioning of implant posts being obliquely inclined and adhering to the implant screws, said screw-in arrangement including a finger wrench comprising a shank (2) with a pocket bore having an outwardly widening cone (3) which, at its inwardly disposed end, turns into a square bore (4), and a knurling wheel (5) at that end of the shank being opposite to the opening of the cone (2), and said screw-in arrangement further including a lever arm wherewith the finger wrench can be actuated in case of non-positive interlock.

2. Screw-in arrangement according to claim 1, characterized in that a central recess (6) is arranged in the top surface of the knurling wheel (5) and several additional recesses (7, 8, ...) are arranged at a regular radial distance from said central recess (6), and in that the lever arm comprises two pegs having a certain distance one from another of which the first peg can be non-positively interlocked with the central recess (6) and the second peg with anyone of the additional recesses (7, 8, ...).

## Revendications

1. Arrangement à visser pour la pose de la vis d'implant dans des os maxillaires et pour le redressement de poteaux d'implant étant obliques et adhérant aux vis d'implantation, ledit arrangement comprenant un clé à doigt comportant une tige (2) avec une forme qui présente un cône s'élargissant vers l'extérieur et se transforment à son bout intérieur en un désage carré (4), et une roue à molet (5) à ce bout de la tige (2) qui est opposé à l'ouverture du cône (3), et ledit arrangement comprenant de plus un bras de levier au moyen duquel on peut actionner ledit clé à doigt à engagement non-positif.

2. Arrangement à visser d'après la revendication 1, caractérisé en ce que, dans la surface supérieure de ladite roue à molet (5), on a disposé un creux central (6) et, à égales distances radiales dudit creux central (6), plusieurs autres creux sont disposés, et que le bras de levier montre deux tenons d'une certaine distance l'un de l'autre dont le premier tenon peut être engagé non-positivement dans le creux central (6) et le second tenon dans un des autres creux (7,8,...).
